# EUROPÄISCHE PATENTSCHRIFT

(11) **EP 0 602 083 B1**
(45) Veröffentlichungstag und Bekanntmachung des Hinweises auf die Patenterteilung: **31.05.1995**
(21) Anmeldenummer: 92917999.2
(22) Anmeldetag: 24.08.1992
(51) Int. Cl.: C07D 209/08, C07D 209/42

(54) **VERFAHREN ZUR HERSTELLUNG VON 5,6-DIHYDROXYINDOLINEN**
PROCESS FOR PRODUCING 5,6-DIHYDROXYINDOLINES
PROCEDE ET FABRICATION DES 5,6-DIHYDROXYINDOLINES

(30) Priorität: 02.09.1991 DE 4129122
(43) Veröffentlichungstag der Anmeldung: 22.06.1994
(73) Patentinhaber: Henkel Kommanditgesellschaft auf Aktien, 40191 Düsseldorf (DE)
(72) Erfinder: KNÜBEL, Georg, D-4000 Düsseldorf 1 (DE); KONRAD, Günther, D-4010 Hilden (DE); MICHEL, Roswitha, D-4000 Düsseldorf 13 (DE)
(86) Internationale Anmeldenummer: EP9201939
(87) Internationale Veröffentlichungsnummer: WO9305017

(56) Entgegenhaltungen:
- DE-A- 2 905 054
- DE-B- 2 908 279
- JOURNAL OF THE CHEMICAL SOCIETY, (C), 1967, Letschworth, GB, Seiten 1424-1427; S.N. MISHRA et al.: "Studies related to the chemistry of melanins. Part III. Synthesis of 5,6-dihydroxyindoline"

## Beschreibung

Die Erfindung betrifft ein Verfahren zur Herstellung von 5,6-Dihydroxyindolinen durch Etherspaltung entsprechender Ethervorstufen mittels Bromwasserstoff und anschließende direkte Auskristallisation aus der wäßrigen Reaktionsmischung.

5,6-Dihydroxyindolin, in der Literatur auch unter den Bezeichnungen Cyclodopamin und Leuconorepinochrom bekannt, sowie 2-Carboxy-5,6-dihydroxyindolin (Synonyme: Cyclodopa und Leucodopachrom) sind im Bereich der Medizin und Pharmazie sowie der Haarfärbung von großer Bedeutung.

Beispielsweise werden die natürlichen Haarfarbstoffe, die sogenannten Melanine, im Verlaufe ihrer Biosynthese durch oxidative Polymerisation von 5,6-Dihydroxyindol gebildet. Es hat daher in der Vergangenheit zahlreiche Versuche gegeben, das 5,6-Dihydroxyindol als reaktives Farbstoffvorprodukt in der Haarfärberei zu verwenden. Leider ist das 5,6-Dihydroxyindol sowohl in freier Form als auch in Form seiner Salze in wäßriger Lösung äußerst instabil und bildet in Gegenwart von Luftsauerstoff sehr rasch unlösliche, gefärbte Oxidations- und Polymerisationsprodukte, die selbst nicht mehr am Haar fixiert werden können. Daher sind Versuche, das 5,6-Dihydroxyindol selbst oder dessen Salze in Färbezubereitungen einzusetzen, mit großen Schwierigkeiten verbunden.

Dagegen ist vorgeschlagen worden, 5,6-Dihydroxyindoline als Pigmentvorstufe in der biomimetischen Färbung von Haaren zu verwenden. Auf diese Weise können natürliche Haarfärbungen mit Melanin-Farbstoffen über ein in situ gebildetes 5,6-Dihydroxyindol erzielt werden, ohne Nachteile durch dessen bekannte Stabilitätsprobleme hinnehmen zu müssen.

Die Herstellung von 5,6-Dihydroxyindolin wurde erstmalig von S.N.Mishra und G.A.Swan beschrieben (J.Chem.Soc. C 1967 1424). Die Autoren erhielten durch Etherspaltung von 5,6-Dimethoxyindolin im Autoklaven bei 150 °C eine Lösung von 5,6-Dihydroxyindolin in Salzsäure. Die Lösung mußte anschließend eingedampft und das so erhaltene Rohprodukt aus Ether/Ethanol gereinigt werden. Dieses Verfahren hat jedoch mehrere Nachteile: (1) die Etherspaltung im Autoklaven ist bei größeren Ansatzmengen mit großem Aufwand verbunden; (2) die Reaktionslösung muß zur Gewinnung des Rohprodukts vollständig eingedampft werden, wodurch erhebliche Energiekosten entstehen und die Raum-Zeit-Ausbeute gemindert wird und (3) die Umkristallisation aus leicht entzündlichen organischen Lösungsmitteln stellt unter dem Aspekt der Arbeitssicherheit ein erhöhtes Risiko dar.

M.Piatelli et al. haben in Kenntnis der Synthese von 5,6-Dihydroxyindolin nach S.N.Mishra und G.A.Swan einen alternativen Syntheseweg entwickelt, bei dem zunächst Dopamin zu Norepichrom oxidiert, dieses zur Leukoverbindung reduziert und dieses in Triacetyl-dihydroxyindolin überführt wird. Aus diesem wird 5,6-Dihydroxyindolin nach Abspaltung der Acetylgruppen in roher Form erhalten. Die Reinigung entspricht der, die S.N. Mishra und G.A.Swan beschrieben haben. Neben der umständlichen Reinigung besitzt auch dieses Verfahren schwerwiegende Nachteile, die einer Anwendung in technischem Maßstab entgegenstehen: (1) Der Oxidationsschritt muß in großer Verdünnung (ca. 0,5 g Dopamin pro Liter) durchgeführt werden und (2) die Zwischenstufe Triacetyldihydroxychinolin muß säulenchromatographisch gereinigt werden.

In völliger Analogie zu diesem Verfahren wurde die Synthese von 2-Carboxy-5,6-Dihydroxyindolin von H.Wyler und J.Chiovini beschrieben (Helv. Chim. Acta 1961 (51) 1476). Sie oxidierten Dopamethylester in großer Verdünnung zum Dopachrom-methylester und reduzierten in situ zum Leucodopachrom-methylester, der dann als Triacetylderivat isoliert und anschließend sauer zu 2-Carboxy-5,6-Dihydroxyindolin (Leucodopachrom) hydrolysiert wurde. Das Verfahren ist jedoch mit den oben beschriebenen Nachteilen verbunden; die Autoren erhielten dementsprechend nur einige Milligramm an Produkt, die zur spektroskopischen Charakterisierung herangezogen wurden.

Gemäß EP-A-462 857 läßt sich 5,6-Dihydroxyindolin durch Umsetzung von 5,6-Dimethoxyindolin mit wäßriger HBr herstellen, wobei nach der Reaktion die Bromwasserstoffsäure abdestilliert, der Rückstand in Ethanol aufgenommen, mit Aktivkohle behandelt und über Celite filtriert wird; anschließend wird zur Auskristallisation von 5,6-Dihydroxyindolin Ethylether zugesetzt. Dieses Verfahren ist für technische Drucke zu umständlich.

Es besteht daher ein Bedarf nach einem verbesserten Verfahren zur Herstellung von 5,6-Dihydroxyindolinen, das insbesondere auch in größerem Maßstab durchführbar ist.

Überraschenderweise wurde nun gefunden, daß sich 5,6-Dihydroxyindoline in einfacher Weise dadurch herstellen lassen, daß man die entsprechenden Ethervorstufen mit Bromwasserstoffsäure umsetzt und die 5,6-Dihydroxyindoline direkt aus der wäßrigen Reaktionsmischung auskristallisiert.

Gegenstand der Erfindung ist daher ein Verfahren zur Herstellung von 5,6-Dihydroxyindolinen der allgemeinen Formel (I) 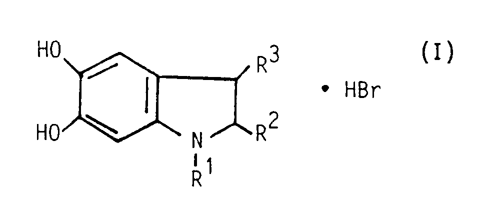
worin die Reste R¹ und R³ unabhängig voneinander Wasserstoff oder Alkylgruppen mit 1 bis 4 C-Atomen und der Rest R² Wasserstoff, eine Alkylgruppe mit 1 bis 4 C-Atomen oder eine Carboxylgruppe bedeuten, durch Umsetzung eines Indolinethers der allgemeinen Formel (II) 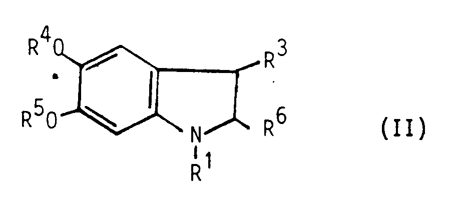
worin die Reste R¹ und R³ unabhängig voneinander Wasserstoff oder Alkylgruppen mit 1 bis 4 C-Atomen, die Reste R⁴ und R⁵ Alkylgruppen mit 1 bis 4 C-Atomen oder gemeinsam mit den Sauerstoffatomen, an die sie gebunden sind, eine Alkylendioxygruppe mit 1 bis 4 C-Atomen und der Rest R⁶ Wasserstoff, eine Alkylgruppe mit 1 bis 4 C-Atomen oder eine Gruppe COOR⁷ oder CONR⁷R⁸ und R⁷ und R⁸ Wasserstoff oder eine Alkylgruppe mit 1 bis 4 C-Atomen bedeuten, mit Bromwasserstoffsäure, wobei die 5,6-Dihydroxyindoline direkt aus der wäßrigen Reaktionsmischung auskristallisiert werden.

In einer bevorzugten Ausführungsform der Erfindung bedeutet der Rest R³ in den Formeln (I) und (II) Wasserstoff. In einer weiteren bevorzugten Ausführungsform bedeutet darüber hinaus der Rest R¹ in den Formeln (I) und (II) Wasserstoff oder eine Methylgruppe, insbesondere Wasserstoff.

Üblicherweise werden in dem erfindungsgemäßen Verfahrens solche Indolinether (II) eingesetzt, bei denen die Reste R⁴ und R⁵ Alkylgruppen mit 1 bis 4 C-Atomen sind. Im Falle von Methylgruppen wird dann im Zuge der Etherspaltung daraus Methylbromid gebildet. Es kann daher vorteilhaft sein, solche Indolinether (II) einzusetzen, bei denen die Reste R⁴ und R⁵ gemeinsam mit den Sauerstoffatomen, an die sie gebunden sind, eine Alkylendioxygruppe mit 1 bis 4 C-Atomen, z.B. eine Methylendioxy- oder Isopropylidendioxygruppe sind. Die in diesem Falll bei der Etherspaltung gebildeten Bromide sind schwerer flüchtig und im Sinne einer besseren Arbeitssicherheit vorzuziehen.

Die eingesetzten Indolinether (II) können dabei in freier Form oder in Form von Salzen, z. B. als Hydrochlorid, eingesetzt werden.

Das erfindungsgemäße Verfahren kann in einfacher Weise derart durchgeführt werden, daß man den Indolinether (II) in einer wäßrigen Lösung von Bromwasserstoff erhitzt. Die Konzentration des wäßrigen HBr unterliegt an sich keiner besonderen Einschränkung, jedoch werden 40 bis 62%-ige Lösungen bevorzugt. Das molare Verhältnis von Bromwasserstoff zu Indolinether (II) wird im Bereich von 3 : 1 bis 30 : 1 eingestellt, vorzugsweise von 5 : 1 bis 15 : 1. Die Reaktionsmischung wird anschließend mehrere Stunden unter Rückfluß erhitzt. Die Aufarbeitung geschieht in einfacher Weise durch Abkühlen des Reaktionsgemisches, wobei das gewünschte 5,6-Dihydroxyindolin (I) auskristallisiert. Nach dem Absaugen und Trocknen fällt das 5,6-Dihydroxyindolin in sehr reiner Form an.

Die nach dem erfindungsgemäßen Verfahren erhaltenen 5,6-Dihydroxyindoline eignen sich als Vorprodukt für Oxidationsfarbstoffe, wie sie in Oxidationsfärbemitteln für Keratinfasern, insbesondere für menschliches Haar, Anwendung finden.

Die folgenden Beispiele dienen der Erläuterung der Erfindung.

### Beispiele

**Beispiel 1**: 100 g 5,6-Dimethoxyindolin (0,6 Mol) wurden unter Stickstoff in einem Rührbehälter vorgelegt und 500 ml einer 62%-igen wäßrigen Bromwasserstofflösung (6,6 Mol HBr) zudosiert. Anschließend wurde vorsichtig erwärmt und das Reaktionsgemisch 5 Stunden unter Rückfluß erhitzt. Nach Abkühlen auf 60 °C wurde filtriert und über Nacht unter Eiskühlung 5,6-Dihydroxyindolin auskristallisiert. Das Produkt wurde abgesaugt und im Vakuum getrocknet.
Ausbeute: 100 g 5,6-Dihydroxyindolin-hydrobromid (0,46 Mol; 78% der Theorie)
Schmelzpunkt: 236-238 °C (Zersetzung)
Reinheit: 97,8% (nach HPLC)
**Vergleichsbeispiel 1**: 10 g 5,6-Dimethoxyindolin wurden unter Stickstoff in einem Rührbehälter vorgelegt und 50 ml konz. HCl zudosiert. Anschließend wurde vorsichtig erwärmt und das Reaktionsgemisch 5 Stunden unter Rückfluß erhitzt. Im Dünnschichtchromatogramm der Reaktionsmischung waren keine Zonen von 5,6-Dimethoxyindolin oder 5,6-Dihydroxyindolin festzustellen.

**Vergleichsbeispiel 2**: 10 g 5,6-Dimethoxyindolin wurden unter Stickstoff in einem Rührbehälter vorgelegt und 50 ml einer 67%-igen wäßrigen Jodwasserstofflösung zudosiert. Anschließend wurde vorsichtig erwärmt und das Reaktionsgemisch 5 Stunden unter Rückfluß erhitzt. Im Dünnschichtchromatogramm der Reaktionsmischung waren keine Zonen von 5,6-Dimethoxyindolin oder 5,6-Dihydroxyindolin festzustellen.

Die Vergleichsbeispiele zeigen deutlich, daß die Herstellung von 5,6-Dihydroxyindolin aus 5,6-Dimethoxyindolin allein mit Bromwasserstoffsäure befriedigend gelingt.

## Patentansprüche

1. Verfahren zur Herstellung von 5,6-Dihydroxyindolinen der allgemeinen Formel (I) 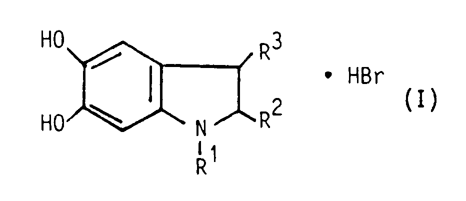 worin die Reste R¹ und R³ unabhängig voneinander Wasserstoff oder Alkylgruppen mit 1 bis 4 C-Atomen und der Rest R² Wasserstoff, eine Alkylgruppe mit 1 bis 4 C-Atomen oder eine Carboxylgruppe bedeuten, durch Umsetzung eines Indolinethers der allgemeinen Formel (II) 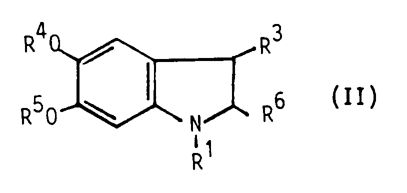 worin die Reste R¹ und R³ unabhängig voneinander Wasserstoff oder Alkylgruppen mit 1 bis 4 C-Atomen, die Reste R⁴ und R⁵ Alkylgruppen mit 1 bis 4 C-Atomen oder gemeinsam mit den Sauerstoffatomen, an die sie gebunden sind, eine Alkylendioxygruppe mit 1 bis 4 C-Atomen und der Rest R⁶ Wasserstoff, eine Alkylgruppe mit 1 bis 4 C-Atomen oder eine Gruppe COOR⁷ oder CONR⁷R⁸ und R⁷ und R⁸ Wasserstoff oder eine Alkylgruppe mit 1 bis 4 C-Atomen bedeuten, mit Bromwasserstoffsäure, wobei die 5,6-Dihydroxyindoline direkt aus der wäßrigen Reaktionsmischung auskristallisiert werden.

2. Verfahren nach Anspruch 1, wobei der Rest R³ Wasserstoff bedeutet.

3. Verfahren nach Anspruch 1 oder 2, wobei der Rest R¹ Wasserstoff oder eine Methylgruppe bedeutet.

4. Verfahren nach einem der Ansprüche 1 bis 3, wobei der Rest R¹ Wasserstoff bedeutet.

## Claims

1. A process for the production of 5,6-dihydroxyindolines corresponding to general formula (I): 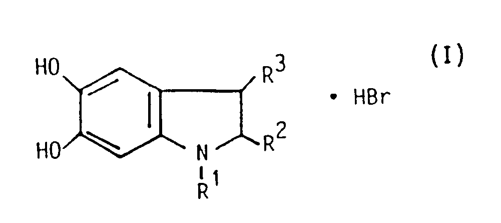 in which R¹ and R³ independently of one another represent hydrogen or C₁₋₄ alkyl groups and R² is hydrogen, a C₁₋₄ alkyl group or a carboxyl group,
by reaction of an indoline ether corresponding to general formula (II): 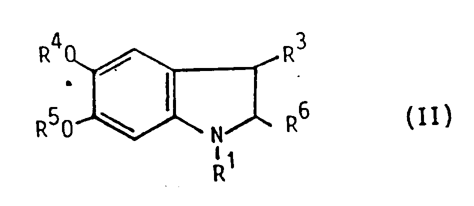 in which R¹ and R³ independently of one another represent hydrogen or C₁₋₄ alkyl groups, R⁴ and R⁵ represent C₁₋₄ alkyl groups or, together with the oxygen atoms to which they are attached, form a C₁₋₄ alkylenedioxy group and R⁶ is hydrogen, a C₁₋₄ alkyl group or a group COOR⁷ or CONR⁷R⁸ and R⁷ and R⁸ are hydrogen or a C₁₋₄ alkyl group,
with hydrobromic acid, the 5,6-dihydroxyindolines being directly crystallized out from the aqueous reaction mixture.

2. A process as claimed in claim 1, R³ being hydrogen.

3. A process as claimed in claim 1 or 2, R¹ being hydrogen or a methyl group.

4. A process as claimed in any of claims 1 to 3, R¹ being hydrogen.

## Revendications

1. Procédé d'obtention de 5,6-dihydroxyindolines de formule générale (I) 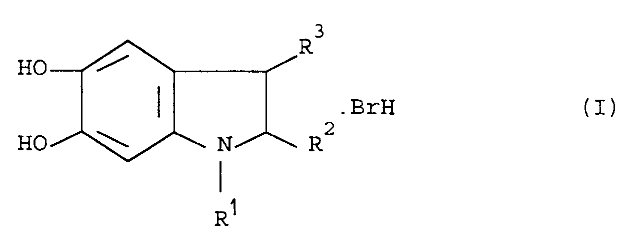 dans laquelle les radicaux R¹ et R³ indépendamment l'un de l'autre, signifient de l'hydrogène ou des groupes alcoyle ayant de 1 à 4 atomes de carbone et le radical R² signifie de l'hydrogène, un groupe alcoyle ayant de 1 à 4 atomes de carbone ou un groupe carboxy
par réaction d'un éther d'indoline de formule générale (II) 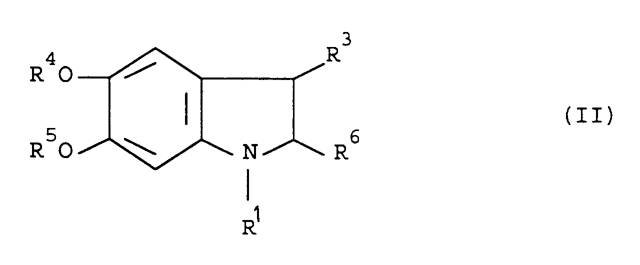 dans laquelle les radicaux R¹ et R³ indépendamment l'un de l'autre signifient de l'hydrogène ou des groupes acoyle ayant de 1 à 4 atomes de carbone
les radicaux R⁴ et R⁵ signifient des groupes alcoyle ayant de 1 à 4 atomes de carbone ou ensemble avec les atomes d'oxygène, auxquels ils sont liés, signifient un groupe alcoylène dioxy ayant de 1 à 4 atomes de carbone et le radical R⁶ signifie un hydrogène, un groupe alcoyle ayant de 1 à 4 atomes de carbone ou un groupe COOR⁷ ou CONR₇R₈ et R⁷ et R⁸ signifient de l'hydrogène ou un groupe alcoyle ayant de 1 à 4 atomes de carbone
avec de l'acide bromhydrique grâce à quoi les 5,6-dihydroxyindolines pouvant être cristallisées directement du mélange réactionnel aqueux.

2. Procédé selon la revendication 1 dans lequel le radical R³ signifie de l'hydrogène.

3. Procédé selon la revendication 1 ou la revendication 2, dans lequel le radical R¹ signifie de l'hydrogène ou un groupe méthyle.

4. Procédé selon l'une des revendications 1 à 3 dans lequel le radical R¹ signifie de l'hydrogène.
